# EUROPÄISCHE PATENTANMELDUNG

(11) **EP 0 980 927 A2**
(43) Veröffentlichungstag der Anmeldung: **23.02.2000**
(21) Anmeldenummer: 99116072.2
(22) Anmeldetag: 16.08.1999
(51) Int. Cl.: D05B 85/02, D05B 85/06, A61B 17/06

(54) **Nähnadel**

(30) Priorität: 17.08.1998 DE 19837144
(71) Anmelder: Bürger, Jürgen, 97453 Schonungen (DE); Derleth, Erich, 97453 Schonungen (DE)
(72) Erfinder: Bürger, Jürgen, 97453 Schonungen (DE); Derleth, Erich, 97453 Schonungen (DE)
(74) Vertreter: Pöhner, Wilfried Anton, Dr.

(57) **Zusammenfassung**

Nähnadel mit einer in der Nähe des hinteren Endes angeordneten Öse zur Aufnahme eines Fadens, welche die Nadel durchgreift, wobei zwischen der Öse (2) und dem seitlichen Nadelrand (5) ein Schlitz (3) verläuft, sowie innerhalb der Öse (2) und/oder durch den Schlitz (3) eine Einschnürung (4a, 4b) gebildet ist.

## Beschreibung

Die Erfindung bezieht sich auf eine Nähnadel mit einer in der Nähe des hinteren Endes angeordneten Öse zur Aufnahme eines Fadens, welche die Nadel durchgreift.

Das Einsatzgebiet von Nähnadeln ist vielseitig. Neben privaten Haushalten verwendet auch die Textilindustrie Nähnadeln für die Verarbeitung von Leder oder Pelzen. Desweiteren sind Nähnadeln in Dienstleistungsbetrieben wie Änderungsschneidereien oder Schuhmacherbetrieben verbreitet. Dazu kommt das Kunstgewerbe des Stickens sowie das Verschließen von Säcken für Zucker, Getreide oder ähnliches. Nicht zuletzt werden Nähnadeln auch in der Medizin zum Verschließen von Wunden eingesetzt. Die bisher bekannten Nähnadeln weisen Ösen auf, in die ein Faden vor der Durchführung von Näharbeiten mühsam eingefädelt werden muß. Diese Tätigkeit stellt nicht nur viele Personen jedesmal neu vor eine Geduldsprobe, sondern erfordert auch Zeit, die bei industriellen Anwendungen mit entsprechenden Kosten verbunden ist und in der Medizin im Einzelfall sogar über Leben und Tod eines Patienten entscheiden kann.

Die Erfindung hat sich demgegenüber die Aufgabe gestellt, eine Nähnadel zu schaffen, in deren Öse sich ein Faden mit weniger Aufwand einführen läßt.

Diese Aufgabe wird erfindungsgemäß dadurch gelöst, daß zwischen der Öse und dem seitlichen Nadelrand ein Schlitz angeordnet ist, sowie innerhalb der Öse und/oder durch den Schlitz eine Einschnürung gebildet ist.

Zum Einführen in die Öse streift der Nutzer mit dem Faden an der mit dem Schlitz versehenen Außenseite der Nadel entlang und zieht den Faden durch den Schlitz in die Öse. Damit beim Nähen der Faden die Öse nicht wieder durch den Schlitz verläßt, ist innerhalb der Öse oder des Schlitzes eine Einschnürung eingebracht. wird Im Sinne der Erfindung wird unter Einschnürung ein Abschnitt des Schlitzes oder der Öse verstanden, der einen geringeren Durchmesser aufweist als die sich beidseitig daran anschließende Abschnitte. In die Öse wird der Faden über die Einschnürung hinweg eingeführt und durch die Einschnürung gehalten.

In vorteilhafter Weiterbildung ist der Durchmesser der Einschnürung wesentlich geringer als der Durchmesser des in Einführungsrichtung dahinter angeordneten Ösenabschnittes. Durch diese geometrische Gegebenheit wird zwar die Möglichkeit, daß der Faden die Öse verläßt, nicht vollkommen ausgeschlossen, jedoch ist die Wahrscheinlichkeit hierfür erheblich verringert, da der Faden zunächst einmal die im Vergleich zum Ösenabschnitt schmale Einschnürung passieren müßte.

Ergänzend kann der Durchmesser der Einschnürung kleiner sein als der Durchmesser des Fadens. Zur Entfernung über die Einschnürung hinweg muß entweder der Faden zusammengedrückt oder die Einschnürung aufgeweitet werden, wozu eine Krafteinwirkung erforderlich ist, die beim Nähen in der Regel nicht auftritt.

Durch das Einfädeln und Durchziehen der Nadel durch das Nähgut wird der Faden in der Öse nach hinten gezogen. Daher ist der Schlitz vorzugsweise nahe am vorderen Ende der Öse angebracht, sodaß der Faden bei jedem Festziehen der Naht aus dem Bereich des Schlitzes entfernt wird. Ein unerwünschtes Verlassen der Öse ist damit zumindest erschwert.

Mit Minimalaufwand läßt sich zwischen Öse und seitlichem Nadelrand ein Schlitz anbringen, der senkrecht zur Längsachse der Nadel verläuft und daher die zur Verbindung von Öse und Nadelrand erforderliche minimale Länge aufweist. Da Nähnadeln in der Regel auch im axialen Bereich der Öse relativ dünn sind, um den zu nähenden Stoff schon bei geringer Krafteinwirkung durchstechen zu können, erstreckt sich die Öse vorzugsweise in axialer Richtung der Nadel, sodaß ein senkrecht zur Längsrichtung der Nadel verlaufender Schlitz auch annähernd senkrecht zur Längsrichtung der Öse ausgerichtet ist. Ein Faden müßte beim Verlassen der Öse seine Bewegungsrichtung relativ zur Nadel genau am Ansatz des Schlitzes ändern, was relativ unwahrscheinlich ist.

Der vom Schlitz ausgehende, in Richtung des hinteren Nadelendes verlaufende Ösenrand, der Schlitz selbst, sowie der vom Schlitz ausgehend in Richtung hinteres Nadelende verlaufende Nadelrand schließen einen Schenkel ein, der sich durch Krafteinwirkung zumindest geringfügig in radialer Richtung elastisch aufbiegen läßt.

Verläuft die dem Schenkel gegenüberliegende, also in Längsrichtung vordere Wand des Schlitzes zur Öse hin mit einer Komponente in Längsrichtung der Nadel nach vorne, so verbreitert sich der Schlitz, wenn bei Einführung des Fadens der Schenkel in Richtung Öseninneres gebogen wird, da sich der an der engsten Stelle des Schlitzes befindliche Punkt des Schenkels in radialer Richtung nach innen, d.h. mit einer Komponente von der gegenüberliegenden Schlitzwand weg bewegt. Bei Krafteinwirkung auf den Schenkel von innen dagegen verengt sich der Schlitz entsprechend. Dadurch wird gleichzeitig die Einführung eines Fadens über den Schlitz in die Öse vereinfacht und ein unerwünschtes Verlassen der Öse über den Schlitz erschwert.

Besteht der beschriebene Schenkel aus elastischem Material, so läßt er sich relativ leicht und ohne bleibende Deformation aufweiten, sodaß der Schlitz auch nach mehrmaliger Benutzung in den Ursprungszustand zurückkehrt. Auch eine Einschnürung in der Öse kann sich durch Krafteinwirkung auf den Schenkel aufweiten, sodaß selbst die Durchführung eines relativ dicken Fadens möglich ist. Kräfte, die beim Nähen auf den Faden gegenüber der Nadel in Richtung vorderes Nadelende wirken und Kräfte, die Einschnürung unerwünschterweise aufweiten könnten, sind dagegen relativ selten und bei geeigneter Auslegung des Schenkels nicht ausreichend, um die erforderliche Aufweitung hervorzurufen.

Die Einführung in die Öse gestaltet sich am einfachsten, wenn der Faden bei Entlangstreifen an dem mit Schlitz versehenen Nadelrand von der Nadelspitze her unter Krafteinwirkung in Richtung des Schlitzes und ohne weiteres Zutun in die Öse fällt und dabei alle Einschnürungen überwindet. Diese Technik des Einfädelns wird ermöglicht, indem die vordere Schlitzwand im umfangsnahen Bereich einen spitzen Winkel mit der anderen Schlitzwand oder der Ösenwand bildet und im spitzen Winkel zur Längsrichtung der Öse mit einer Komponente in Richtung hinteres Nadelende verläuft. Beim Entlangstreifen an der Nadel erreicht der Faden zunächst den umfangsnahen vorderen Schlitzrand, an dem er sich entlangbewegt. Nach einem weiteren zurückgelegten Wegstück trifft der Faden auf die andere Schlitzwand oder eine Ösenwand, streift dort entlang und bewegt sich schließlich in Längsrichtung der Öse, wo er ggf. noch Einschnürungen passieren kann.

Im Bereich einer Einschnürung verläuft die Öse vorzugsweise in Längsrichtung der Nadel. Dadurch wird erreicht, daß ein Faden, der beim Nähen unbeabsichtigterweise passiert, beim nächsten Durchziehen der Nadel die Einschnürung über einen in Gegenrichtung passiert und in den gewünschten Teil der Öse zurückgezogen wird.

In vorteilhafter Ausbildung einer Nadel mit Einschnürung in der Öse verläuft der dem Schlitz gegenüberliegende seitliche Ösenrand in Längsrichtung der Öse bis zur engsten Stelle der Einschnürung ohne Knick und im wesentlichen gerade. An diesem Ösenrand kann der Faden zur Überwindung der Einschnürung kontrolliert und dauerhaft anliegend entlangbewegt werden, ohne die Gefahr des Verlassens der Öse über den Schlitz.

Vorzugsweise verläuft der vom Schlitz abgewandte seitliche Ösenrand über die Einschnürung hinweg in Längsrichtung der Nadel und läßt sich damit über eine längere Strecke hinweg nahe am Nadelrand vorbeiführen, sodaß die Nadel auch im axialen Bereich der Öse mit einer minimalen Dicke gebildet sein kann, was das Durchstechen des Nähgutes erleichter.

Einschnürungen sollten erlauben einen Faden von außen mit möglichst wenig Kraftaufwand und Mühe hindurchzuführen. Dies wird erreicht, indem Einschnürungen in Einführungsrichtung vor der engsten Stelle zumindst abschnittsweise konisch zulaufen und damit den Faden in der gewünschten Richtung führen.

Die konisch zulaufenden Abschnitte sollten möglichst lang sein, also idealerweise von der am weitesten davorliegenden Aufweitung bis zur engsten Stelle der Einschnürung. Im Falle einer Einschnürung im Schlitz befindet sich die weiteste Stelle idealerweise am Schlitzrand, bei einer Einschnürung der Öse an der weitesten Stelle des in Einführungsrichtung vor der Einschnürung gelegenen Ösenabschnitts.

In Gegenrichtung allerdings sollte ein Passieren der Einschnürung erschwert sein. Unerwünschte konische Führungen zur engsten Stelle der Einschnürung hin werden vermieden, indem sich die Einschnürung an ihrer engsten Stelle unvermittelt aufweitet.

Einige Nähnadeln sind zur besseren Handhabbarkeit in Längsrichtung mit einer Krümmung versehen. Die Wahrscheinlichkeit, daß der Faden den Schlitz unbeabsichtigt von der Öse ausgehend passiert, läßt sich reduzieren, indem der Schlitz an der krümmungsinneren Seite angebracht ist.

Weitere Einzelheiten, Merkmale und Vorteile der Erfindung lassen sich dem nachfolgenden Beschreibungsteil entnehmen, in dem anhand von Zeichnungen Ausführungsbeispiele der Erfindung näher erläutert sind. Dabei zeigen
- **Figur 1**: die Gesamtdarstellung einer Nadel und
- **Figuren 2 - 6**: unterschiedliche Formen der Ösen erfindungsgemäßer Nadeln im Detail

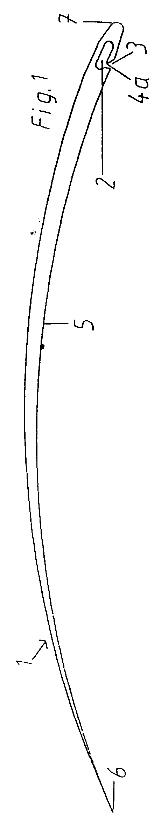

Figur 1 stellt eine Nadel (1) vollständig dar, die in diesem Spezialfall in Längsrichtung zwischen der Spitze (6) und dem hinteren Ende (7) gekrümmt ist. Nahe am hinteren Ende (7) ist die Nadel (1) mit einer Öse (2) versehen, die mit dem krümmungsinneren Nadelrand (5) über einen Schlitz (3), durch den sich ein Faden einführen läßt, verbunden ist. Um zu verhindern, daß der Faden beim Nähen die Öse (2) über den Schlitz (3) verläßt, ist eine Einschnürung (4a) bei dieser speziellen Ausgestaltung durch den Schlitz (3) gebildet, vorhanden.

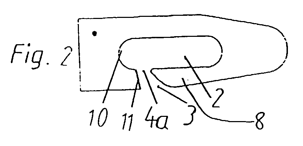

Figur 2 stellt die Öse (2) der in Figur 1 beschriebenen Nadel (1) im Detail dar. Da der Schlitz (3) zwar nicht vollständig, aber doch annähernd senkrecht zum Nadelrand verläuft, ist die durch ihn zu überbrückende Strecke relativ kurz. Die Öse (2) wird vom Schlitz (3) in der Nähe ihres vorderen Endes (10) erreicht, sodaß der Faden während des Nähens durch das sich wiederholende Durchstechen innerhalb der Öse in Längsrichtung der Nadel nach hinten, und damit vom Schlitz weggezogen wird. Die Einschnürung (4a) ist im Schlitz (3) vorhanden, der vom Nadelrand ausgehend bis zur engsten Stelle der Einschnürung konisch zuläuft und damit die Einführung eines Fadens erheblich vereinfacht. Innen dagegen weitet sich die Einschnürung (4a) unvermittelt auf, sodaß dort keine konische Führung vorhanden ist, die eine Überwindung der Einschnürung (4a) seitens der Öse begünstigen würde, sodaß der Faden relativ sicher in der Öse (2) gehalten wird. Öse (2), Schlitz (3) sowie seitlicher Nadelrand begrenzen einen Schenkel (8), der federnde Eigenschaften besitzt und dessen Spitze sich bei Einführung des Fadens in radialer Richtung nach innen bewegt und sich damit vom vorderen Schlitzrand (11) entfernt, was eine Aufweitung der Einschnürung (4a) bedeutet, während bei Entfernung des Fadens aus der Öse (2) sich die Einschnürung (4a) verengt. Damit wird nicht nur die Einführung des Fadens in die Öse (2) begünstigt, sondern auch der Rückhalt verbessert.

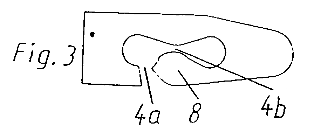

Die in Figur 3 dargestellte Öse weist gegenüber Figur 2 eine zusätzlich Einschnürung (4b) im Bereich der Öse auf, die durch ein konisches Zulaufen von außen her die Einführung eines Fadens vereinfacht. Bei Passieren des konischen Abschnittes durch den Faden kann sich der Schenkel (8) verbiegen, sodaß sich die Einschnürung (4b) aufweitet und damit auch einen Faden passieren läßt, der deutlich dicker ist als die engste Stelle der Einschnürung. Im Gegensatz zu der in Figur 2 dargestellten, aus deren Beschreibung sich weitere Merkmale der hier beschriebenen Öse entnehmen lassen, weiten sich die Einschnürungen nicht unvermittelt an der engsten Stelle auf, was den unerwünschten Effekt hat, daß ein Faden leichter beim Nähen eine Einschnürung (4a,b) entgegengesetzt zur Einführungsrichtung passieren kann. Allerdings muß der Faden zum Verlassen der Öse zwei Einschnürungen (4b, 4a) passieren, was relativ unwahrscheinlich erscheint, zumal die hintere Einschnürung (4b) in Längsrichtung der Öse verläuft, sodaß ein Faden nach unbeabsichtigten Passieren beim nächsten Durchstechen der Nadel wieder zurück durch diese Einschnürung (4b) geführt wird.

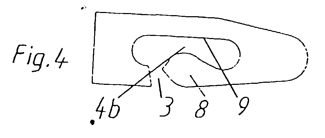

Figur 4 stellt eine Öse dar, die bis auf die hintere Einschnürung (4b) der in Figur 3 dargestellten entspricht. Der Unterschied besteht darin, daß sich die Öse lediglich von einem seitlichen Rand her einengt, weshalb die Einschnürung (4b) auch eine größere Breite hat. Dagegen verläuft der dem Schlitz (3) gegenüberliegende Ösenrand (9) über die Einschnürung (4b) hinweg ohne Knick und nennenswerte Krümmung, wodurch beim Einführen der Nadel das Passieren der Einschnürung (4b) vereinfacht ist, indem der Faden an diesem Ösenrand (9) entlanggeführt wird. Da der Ösenrand (9) dabei in Längsrichtung der Nadel verläuft, läßt sich die Nadel im Bereich der Öse in minimaler Dicke bilden, was das Durchstechen des Nähgutes zu nähenden vereinfacht.

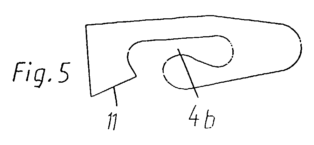

Figur 5 stellt eine Öse dar, die der in Figur 3 gezeigten ähnlich ist. Der Unterschied besteht darin, daß der vordere Schlitzrand (11) auf einem vom Nadelrand (5) ausgehenden Stück schräg in Richtung hinteres Nadelende (7) verläuft und damit das Einführen eines Fadens durch Entlangstreifen am seitlichen Nadelrand (5) von der Spitze (6) kommend ermöglicht. Mit Erreichen des vorderen Schlitzrandes (11) bewegt sich der Faden zunächst daran ein Stück entlang, verliert den Schlitzrand (11) und trifft bei Weiterführung auf den gegenüberliegenden Schlitzrand oder einen Ösenrand, durchläuft schließlich in der Öse (2) die Einschnürung (4b) und gelangt bis zum hinteren Ende.

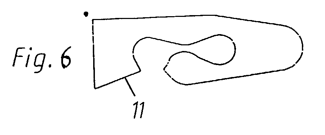

Bei der in Figur 6 dargestellten Öse weist der vordere Schlitzrand (11) die gleiche Form auf, wie in Figur 5, was mit den entsprechenden Vorteilen bei der Einführung eines Fadens verbunden ist. Die Ösenform entspricht dagegen der von Figur 4.

Insgesamt erhält man eine Nähnadel, in deren Öse sich ein Faden über einen Schlitz mühelos einführen läßt und bei der durch Einschnürungen im Bereich der Öse oder des Schlitzes verhindert wird, daß der Faden beim Nähen die Öse unbeabsichtigterweise verläßt.

## Patentansprüche

1. Nähnadel mit einer in der Nähe des hinteren Endes angeordneten Öse zur Aufnahme eines Fadens, welche die Nadel durchgreift,
**dadurch gekennzeichnet,** daß
- zwischen der Öse (2) und dem seitlichen Nadelrand (5) ein Schlitz (3) verläuft, sowie
- innerhalb der Öse (2) und/oder durch den Schlitz (3) eine Einschnürung (4a, 4b) gebildet ist.

2. Nähnadel nach Anspruch 1, **dadurch gekennzeichnet,** daß der minimale Durchmesser der Einschnürung (4a, 4b) wesentlich geringer ist als der Durchmesser des hinter der Einschnürung (4a, 4b) angeordneten Ösenabschnittes.

3. Nähnadel nach Anspruch 1 oder 2, **dadurch gekennzeichnet**, daß der minimale Durchmesser der Einschnürung (4a, 4b) kleiner ist als der Durchmesser des Fadens.

4. Nähnadel nach einem der vorhergehenden Ansprüche, **dadurch gekennzeichnet,** daß der Schlitz (3) mit der Öse (2) nahe an deren vorderen Ende (10) verbunden ist.

5. Nähnadel nach einem der vorhergehenden Ansprüche, **dadurch gekennzeichnet,** daß der Schlitz (3) annähernd senkrecht zur Längsrichtung der Nadel verläuft.

6. Nähnadel nach einem der vorhergehenden Ansprüche, **dadurch gekennzeichnet,** daß die in Längsrichtung der Nadel vordere Wand (11) des Schlitzes an der engsten Stelle des Schlitzes zur Öse hin mit einer Komponente in Längsrichtung der Nadel nach vorne verläuft.

7. Nähnadel nach einem der vorhergehenden Ansprüche, **dadurch gekennzeichnet,** daß der durch Öse (2), Schlitz (3) und vom Schlitz in Richtung hinteres Nadelende (7) verlaufenden Nadelrand (5) eingeschlossene Schenkel (8) aus elastischem Material gebildet ist.

8. Nähnadel nach einem der vorhergehenden Ansprüche, **dadurch gekennzeichnet,** daß
- der umfangsnahe Bereich des Schlitzrandes (11) einen spitzen Winkel mit der gegenüberliegenden Schlitzrand oder einen Ösenrand einschließt und
- im spitzen Winkel zur Längsrichtung der Öse (2) mit einer Komponente in Richtung hinteres Nadelende (7) verläuft.

9. Nähnadel nach einem der vorhergehenden Ansprüche mit einer Einschnürung in der Öse, **dadurch gekennzeichnet,** daß die Öse (2) im Bereich der Einschnürung (4b) im wesentlichen in Längsrichtung der Nadel verläuft.

10. Nähnadel nach einem der vorhergehenden Ansprüche mit einer Einschnürung in der Öse, **dadurch gekennzeichnet,** daß der vom Schlitz (3) abgewandte seitliche Ösenrand (9) in Längsrichtung der Öse (2) bis zur engsten Stelle der Einschnürung (4b) ohne Knick und im wesentlichen gerade verläuft.

11. Nähnadel nach Anspruch 10, **dadurch gekennzeichnet,** daß der vom Schlitz (3) abgewandte seitliche Ösenrand (9) über die Einschnürung (4b) hinweg in Längsrichtung der Nadel (1) verläuft.

12. Nähnadel nach einem der vorhergehenden Ansprüche, **dadurch gekennzeichnet,** daß der Schlitz (3) in Richtung Öse (2) und/oder die Einschnürung (4b) der Öse (2) in deren Längsrichtung nach hinten zumindest abschnittsweise konisch zuläuft.

13. Nähnadel nach Anspruch 12, **dadurch gekennzeichnet,** daß der Schlitz (3) vom Nadelrand (5) ausgehend bis zur engsten Stelle bzw. die Öse (2) von der weitesten Stelle des vorderen Abschnitts bis zur engsten Stelle der Einschnürung (4b) konisch zuläuft.

14. Nähnadel nach einem der vorhergehenden Ansprüche, **dadurch gekennzeichnet,** daß sich die Einschnürung (4a bwz. 4b) zur Öse (2) bzw. zum hinteren Abschnitt der Öse (2) hin an der engsten Stelle unvermittelt aufweitet.

15. Nähnadel nach einem der vorhergehenden Ansprüche, die in Längsrichtung eine Krümmung aufweist, **dadurch gekennzeichnet,** daß sich der Schlitz (3) am krümmungsinneren Nadelrand (5) befindet.
